# EUROPEAN PATENT APPLICATION

(11) **EP 4 461 343 A1**
(43) Date of publication of application: **13.11.2024**
(21) Application number: 23737046.5
(22) Date of filing: 04.01.2023
(51) Int. Cl.: A61N 1/36, A61N 5/06, A61N 5/00, A61B 18/18, A61B 5/00

(54) **CONTROL SYSTEM OF BEAUTY INSTRUMENT, AND BEAUTY INSTRUMENT**

(30) Priority: 07.01.2022 CN 202210018267
(71) Applicant: Xiamen Solex High-Tech Industries Co., Ltd., Xiamen, Fujian 361028 (CN)
(72) Inventor: JIN, Chenying, Xiamen, Fujian 361028 (CN); LIU, Tingke, Xiamen, Fujian 361028 (CN); WEI, Fan, Xiamen, Fujian 361028 (CN); LIN, Caigui, Xiamen, Fujian 361028 (CN)
(74) Representative: Verscht, Thomas Kurt Albert
(86) International application number: PCT/CN2023/070441
(87) International publication number: WO 2023/131183

(57) **Abstract**

Disclosed are a control system for a beauty instrument, and a beauty instrument. The system comprises: a motion acquisition module and a control module; the motion acquisition module is used for converting a movement signal during working of a beauty instrument into an electrical signal; the control module is connected to the motion acquisition module, receives the electrical signal and performs analysis to obtain movement data of the beauty instrument, and controls an output gear and/or a working mode of the beauty instrument on the basis of the movement data. According to the present invention, the change of a motion state of the beauty instrument can be detected, the output gear and/or the working mode can be adjusted, and the user experience is improved.

## Description

### FIELD OF THE DISCLOSURE

The present disclosure relates to the technique field of personal care products, and in particular is a control system of a beauty device and the beauty device.

### BACKGROUND OF THE DISCLOSURE

With the progress and development of society, beauty pursuit of person is getting higher and higher, various beauty care devices are also emerged accordingly in view of love of beauty from person and widely accepted by person. With a rapid popularization of the beauty devices, requirements of person for the beauty devices are also higher and higher, the conventional beauty devices can merely output by choosing a certain fixed gear position and cannot be automatically adjusted according to a way of use, which is not smart enough. Therefore, when beauty care is performed at a single point, intensity is too large, when repeated movement operation is performed at a section, an application area is too large, resulting in intensity of the body feeling being too small, that is, a moving state cannot be recognized so as to be automatically adjusted to ensure a good experience.

In addition, beauty care of products of the beauty devices on the market selects a designated gear position basically through the button, and output cannot be automatically adjusted according to the way of use, which is not smart enough. With respect to a small part of the beauty devices, multi-axis acceleration or a gyroscope sensors (3-axis, 6-axis, etc.) are used to identify a movement (an acceleration sensor identifies variations in acceleration, a gyroscope sensor identifies variations in inclination), however, when it moves at a fixed inclination angle at a constant speed during use, a current movement state cannot be identified by the acceleration sensor.

### BRIEF SUMMARY OF THE DISCLOSURE

The main objective of the present disclosure is to provide a control system of a beauty device and the beauty device, which overcomes the deficiencies of the beauty device in the existing techniques, detects variations in a movement state of the beauty device, adjusts an output gear position and/or a working mode, and improves the user experience.

A technical solution of the present disclosure is as follows:
A control system of a beauty device, comprising: a movement acquisition module and a control module; the movement acquisition module is used to convert a movement signal of the beauty device during work into an electrical signal; the control module is connected to the movement acquisition module, receives the electrical signal, obtains movement data of the beauty device by analyzing, and controls an output gear position and/or a working mode of the beauty device based on the movement data.

Preferably, the electrical signal comprises a pulse signal; the movement acquisition module is used to convert the movement signal of the beauty device during work into the electrical signal, specifically comprising:
The movement acquisition module is used to convert the movement signal of the beauty device during work into a rolling signal and output the pulse signal based on the rolling signal.

Preferably, the movement acquisition module comprises one or more balls, one or more rollers and one or more rotary encoders; the one or more balls are disposed in a working head of the beauty device and are partially exposed on a contact surface with a skin; the one or more rollers are connected to the one or more balls by contacting so as to rotate when the one or more balls roll; the one or more rollers are coaxially connected to the one or more rotary encoders; the one or more rotary encoders are electrically connected to the control module to transmit the pulse signal; the control module receives the pulse signal to control the output gear position and/or the working mode of the beauty device.

Preferably, the working head comprises a first opening for exposing the one or more balls.

Preferably, the one or more rollers comprise an X-axis roller for detecting a movement of the one or more balls in an X-axis and a Y-axis roller for detecting a movement of the one or more balls in a Y-axis; the one or more rotary encoders comprise an X-axis rotary encoder and a Y-axis rotary encoder; the X-axis roller is connected to the X-axis rotary encoder; the Y-axis roller is connected to the Y-axis rotary encoder; the X-axis rotary encoder and Y-axis rotary encoder are respectively electrically connected to the control module.

Preferably, the movement acquisition module further comprises an accommodating cavity; the one or more balls are disposed in the accommodating cavity.

Preferably, the one or more balls disposed in the accommodating cavity comprise one or more; when two or more are included, all of the one or more balls are disposed by following a direction of a bottom of the accommodating cavity in sequence.

Preferably, a spring is disposed on the bottom of the accommodating cavity; the spring is compressed by the one or more balls in the accommodating cavity.

Preferably, a side of the accommodating cavity comprises one or more second openings; the one or more balls are in close contact with the one or more rollers through the one or more second openings.

Preferably, an outer side of the accommodation cavity is disposed with lugs for securing the one or more rollers.

Preferably, the control module receives the pulse signal, obtains the movement data of the beauty device by analyzing, and controls the output gear position of the beauty device based on the movement data, specifically comprising:
The control module receives the pulse signal and detects a number of received pulses within a preset time;
The number of the received pulses is converted into a moving distance, the moving distance is divided by the preset time to obtain a moving speed of the one or more balls;
The output gear position of the beauty device is adjusted based on the moving speed of the one or more balls.

Preferably, the control module receives the pulse signal, obtains the movement data of the beauty device by analyzing, and controls the working mode of the beauty device based on the movement data, specifically comprising:
The control module receives the pulse signal and detects a number of pulses in the X-axis and a number of pulses in the Y-axis received within a preset time;
The number of the pulses in the X-axis is converted into a moving distance in the X-axis; the pulse number in the Y-axis is converted into a moving distance in the Y-axis;
A moving path of the one or more balls is detected based on the moving distance in the X-axis and the moving distance in the Y-axis;
The working mode of the beauty device is adjusted based on the moving path of the one or more balls.

On the other hand, a beauty device, comprising a handheld body and a working head; it further comprises the control system of the beauty device; the movement acquisition module is disposed in the working head.

Preferably, the beauty device comprises a facial beauty device.

Preferably, the beauty device comprises a hair removal device.

Compared with the existing techniques, the present disclosure has the following advantages:
(1) An inner portion of the working head of the beauty device of the present disclosure is disposed with a ball that is partially exposed on a contact surface with a skin and a roller in close contact with a surface of the ball, when the beauty device moves while fitting the skin, a friction force generated between the ball and the skin drives the ball to roll, when the ball rotates, a generated friction force drives the roller to rotate in accordance with a rolling direction, so as to drive the rotary encoder connected to a rear end of the roller to rotate together, the rotary encoder transmits the pulse signal to the control module, and the control module detects the moving speed and/or the moving path of the beauty device, controls the output gear position and/or the working mode of the beauty device according to the moving speed and/or the moving path, so as to improve the user experience;
(2) The present disclosure comprises the accommodating cavity for accommodating the ball, one or more of the balls can be fixed in the accommodating cavity, so that the generated friction force between the ball and the skin can drive the ball to roll when the beauty device moves while fitting the skin; the spring abutting the ball is disposed on the bottom of the accommodating cavity to further be tightly pressed by the ball; the side of the accommodating cavity comprises an opening and the outer side of the accommodating cavity is disposed with lugs for securing the roller, so that the ball is in close contact with the roller (the X-axis or Y-axis rollers), thus driving the encoder to rotate and ensuring force transmission;
(3) The control module of the present disclosure can detect the moving speed and/or the moving path of the ball (the beauty device) based on the pulse signal output by the rotary encoder, and the output gear position is automatically adjusted according to the moving speed, so as to obtain same or similar body feelings when beauty care is performed at a single point, when the beauty care is performed during a rapid movement and when the beauty care is performed in a section; furthermore, with respect to the beauty device with multiple working modes, and the working modes can be automatically adjusted according to the moving path.

The foregoing description is merely a summary of the technical solution of the present disclosure, and specific embodiments of the present disclosure are illustrated below in order to enable the technical methods of the present disclosure to be more clearly understood so as to be implemented in accordance with the specification and to enable the aforementioned and other objectives, features and advantages of the present disclosure to be more apparent and understandable.

The specific embodiments of the present disclosure are specifically described in combination with the following description and the accompanying drawings, and the aforementioned and other objectives, the advantages and the features of the present disclosure will be more apparent to technical persons skilled in the art.

### BRIEF DESCRIPTION OF THE DRAWINGS

FIG. 1 shows a structural diagram of a control system of Embodiment 1 of the present disclosure;
FIG. 2 shows a sectional view of a movement acquisition module of the control system of Embodiment 1 of the present disclosure;
FIG. 3 shows a perspective view of the movement acquisition module of the control system of Embodiment 1 of the present disclosure;
FIG. 4 shows a structural diagram of a control system for a facial beauty device of Embodiment 2 of the present disclosure;
FIG. 5 shows a diagrammatic view of an overall structure of the facial beauty device of Embodiment 2 of the present disclosure;
FIG. 6 shows a sectional view of the facial beauty device of Embodiment 2 of the present disclosure;
FIG. 7 shows an exploded perspective view of the facial beauty device of Embodiment 2 of the present disclosure;
FIG. 8 shows an exploded perspective view of main components within an working head of the facial beauty device of Embodiment 2 of the present disclosure;
FIG. 9 shows a circuit diagram of the control system of the facial beauty device of Embodiment 2 of the present disclosure; wherein (a) shows a circuit diagram of an input of a rotary encoder; (b) shows a circuit diagram of an MCU controller; and (c) shows a circuit diagram of an output of a radiofrequency;
FIG. 10 shows a diagrammatic view of an overall structure of a hair removal device of Embodiment 3 of the present disclosure.

### DETAILED DESCRIPTION OF THE EMBODIMENTS

The technical solutions in the embodiments of the present disclosure will be clearly and completely described in combination with the accompanying drawings in the embodiments of the present disclosure; it is obvious that the described embodiments are some embodiments of the present disclosure instead of all embodiments, all other embodiments fall within the protection scope of the present disclosure provided that they are obtained by ordinary technical persons skilled in the art based on the embodiments of the present disclosure without creative works.

In the description of the present disclosure, it should be noted that the terms "including", "comprising" or any other variations thereof are intended to cover non-exclusive inclusion, so that a process, a method, a product or a device including a series of elements not only comprises these elements, but also comprises other elements not expressly listed, or comprises elements inherent to the process, the method, the product or the device. Without further limitation, an element limited by a sentence "comprising a ......" does not exclude an existence of another identical element in the process, the method, the product or the device comprising the element.

In the description of the present disclosure, it should be noted that the terms, such as "upper", "lower", "inner", "outer", "top/bottom", indicate orientations or positional relationships based on orientations and positional relationships shown in the drawings, so as to easily describe the present disclosure and simplify the description, rather than indicating or implying that the referenced or implied device or element should have a specific orientation or be constructed and operated in a specific orientation and therefore should not be understood as a limitation of the present disclosure. Further, the terms "first" and "second" are merely used for description and can not to be understood as indicating or implying relative importance.

In the description of the present disclosure, unless otherwise specified and limited, it should be noted that the terms, such as "installed", "disposed", "sleeved/socketed", "connected", should develop broad understanding, for example, "connected" can be a fixed connection, a detachable connection, or an integrated connection, a mechanical connection, an electrical connection, a direct connection, an indirect connection through an intermediator, or communication between inner portions of two members, for ordinary technical person skilled in the art, specific meaning of the terms in the present disclosure can be understood under specific conditions.

### Embodiment 1

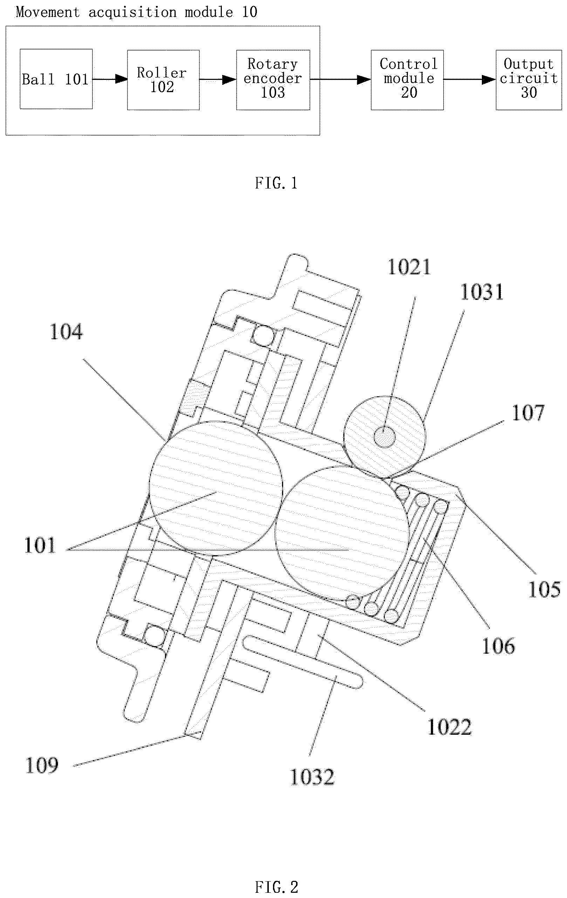

Referring to FIGS. 1-3, a control system of a beauty device, it comprises: a movement acquisition module 10 and a control module 20; the movement acquisition module 10 comprises one or more balls 101, one or more rollers 102 and one or more rotary encoders 103; the one or more balls 101 are disposed in a working head of the beauty device and are partially exposed on a contact surface with a skin; the one or more rollers 102 are connected to the one or more balls 101 by contacting so as to rotate when the one or more balls 101 roll; the one or more rollers 102 are coaxially connected to the one or more rotary encoders 103; the one or more rotary encoders 103 are electrically connected to the control module 20 to transmit pulse signals; the control module 20 receives the pulse signals to control an output circuit 30 of the beauty device to adjust output gear positions and/or working modes.

In this embodiment, the control module 20 comprises an MCU controller, the MCU controller is disposed on a main circuit board. The one or more rotary encoders 103 are electrically connected to the MCU controller to transmit the pulse signals; the MCU controller receives the pulse signals to control the output gear positions and/or the working modes of the beauty device. The one or more rotary encoders 103 comprise an optical encoder, a magnetic encoder, etc.

In this embodiment, the working head comprises a first opening 104 for exposing the one or more balls 101. In this embodiment, the one or more rollers 102 comprise an X-axis roller 1021 for detecting an X-axis movement of the one or more balls 101 and a Y-axis roller 1022 for detecting a Y-axis movement of the one or more balls 101; the one or more rotary encoders 103 comprise an X-axis rotary encoder 1031 and a Y-axis rotary encoder 1032; the X-axis roller 1021 is connected to the X-axis rotary encoder 1031; the Y-axis roller 1022 is connected to the Y-axis rotary encoder 1032; the X-axis rotary encoder 1031 and Y-axis rotary encoder 1032 are connected to the control module 20 respectively.

In this embodiment, the movement acquisition module 10 further comprises an accommodating chamber 105 for securing the one or more balls; the one or more balls 101 are disposed in the accommodating chamber 105, and upper and lower ends of the one or more balls 101 can tightly fit two sides of the accommodating chamber 105 for clamping, and a front end of the one or more balls 101 partially protrudes out of the first opening 104.

Further, in order to enable the one or more balls 101 to cooperate with a size of the accommodating cavity 105, one, two and more of the one or more balls 101 can be disposed in the accommodating cavity 105. All of the one or more balls 101 are disposed by following a direction of a bottom of the accommodating cavity 105 in sequence and are tightly fitted each other when two and more are included.

In order to further secure the one or more balls 101 in the accommodating cavity 105, a spring 106 is disposed on the bottom of the accommodating cavity 105 in this embodiment; the spring 106 is compressed by the one or more balls 101 in the accommodating cavity 105 to enable the one or more balls 101 to be secured in the accommodating cavity 105.

In order to enable the one or more rollers 102 to tightly contact the one or more balls 101, one or more sides of the accommodation cavity 105 of this embodiment comprises one or more second openings 107 to expose a portion of the one or more balls 101; the one or more balls 101 are in close contact with the one or more rollers 102 through the one or more second openings 107, and when the one or more balls 101 rotate, generated friction forces drive the one or more rollers 102 (the X-axis or Y-axis rollers 102) to rotate according to a rolling direction.

In order to enable the one or more rollers 102 to tightly contact the one or more balls 101, an outer side of the accommodating cavity 105 comprises lugs 108 for securing the one or more rollers 102. Specifically, the one or more rollers 102 are erected between the lugs 108.

In order to further secure the one or more rollers 102 and the one or more rotary encoders 103 and to enable the pulse signals of the one or more rotary encoders 103 to be transmitted to the control module 20, this embodiment also comprises a PCB board 109 for opto-electronic signals. The PCB board is sleeved on the accommodating cavity 105 and is disposed with a stopping block 110 for preventing the one or more rotary encoders 103 from deviation.

In this embodiment, the control module 20 receives the pulse signals, controls the output gear positions of the beauty device, and specifically comprises:
The control module 20 receives the pulse signals and detects a number of received pulses within a preset time;
The number of the received pulses is converted into a moving distance, the moving distance is divided by the preset time to obtain a moving speed of the one or more balls 101;
The output gear positions of the beauty device are adjusted based on the moving speed of the one or more balls 101.

An example for obtaining the moving speed is as follows:
The one or more rotary encoders 103 are one or more dual output rotary encoders 103, i.e. the one or more rotary encoders 103 output two pulse signals with a phase difference of 90 degrees. An A-phase pulse is ahead of a B-phase pulse during a clockwise rotation, and the B-phase pulse is ahead of the A-phase pulse during a counterclockwise rotation. Therefore, a rotation direction can be determined.

Assuming that the one or more rotary encoders 103 output 100 pulses by rotating one revolution (360°), the control module 20 can obtain a rotation angle by a number of captured pulses. In addition, assuming that the one or more rotary encoders 103 rotate one revolution, the moving distance is 1 CM, the control module 20 can convert the number of the received pulses into the moving distance, at the same time, the control module 20 records a time for capturing pulses, and if the 100 pulses are captured in 1S, the speed is 1 CM/S.

Specifically, when the control module 20 recognizes that the moving speed of the one or more balls 101 increases and is greater than a preset value or lasts a preset time, which indicates that the beauty device is moving rapidly, the output gear positions of the beauty device can be automatically adjusted to a higher gear position, and no further adjustment is performed when the highest gear position is reached; when the control module 20 recognizes that the moving speed of the one or more balls 101 slows down and is less than the preset value or lasts the preset time, which indicates that the beauty device is decelerated, the output gear positions of the beauty device can be automatically adjusted to a lower gear position; when the control module 20 recognizes that the moving speed of the one or more balls 101 is 0 and lasts the preset time, the gear positions can be automatically adjusted to the lowest gear position, further, when it recognizes that the moving speed of the one or more balls 101 is 0 and lasts another preset time more than the preset time, in order to prevent a motor from burning out due to blocking of the working head 1, a power supply can be automatically shut down and prompted.

With respect to the beauty device having multiple working modes, the control module 20 receives the pulse signals, controls the working modes of the beauty device, specifically comprises:
The control module 20 receives the pulse signals and detects a number of pulses in the X-axis and a number of pulses in the Y-axis received within the preset time;
The number of the pulses in the X-axis is converted into the moving distance in the X-axis; and the number of the pulses in the Y-axis is converted into the moving distance in the Y-axis;
A moving path of the one or more balls 101 is detected based on the moving distance in the X-axis and the moving distance in the Y-axis;
The working modes of the beauty device are adjusted based on the moving path of the one or more balls.

An example for obtaining the moving path is as follows:
If a ratio of the moving distance in the X-axis and the moving distance in the Y-axis shows a linear variation with a relatively fixed slope, the moving path is judged to be a straight movement; if the moving distance in the X-axis and the moving distance in the Y-axis define alternate increasing and decreasing variations, the moving path is judged to be a circular movement.

An inner portion of the working head 1 of the beauty device of the present disclosure is disposed with the one or more balls 101 partially exposed on a contact surface with a skin and the one or more rollers 102 in close contact with a surface of the one or more balls 101, when the beauty device moves while fitting the skin, the friction force generated between the one or more balls 101 and the skin drives the one or more balls 101 to roll, when the one or more ball 101 rotate, the generated friction force drives the one or more rollers 102 (the X-axis or Y-axis rollers 102) to rotate in accordance with a rolling direction, thereby driving the one or more rotary encoders 103 connected to one or more rear ends of the one or more rollers 102 rotating together, the one or more rotary encoders 103 transmit the pulse signals to the control module 20, the control module 20 detects the moving speed and/or the moving path of the beauty device, controls the output gear positions and/or the working mods of the beauty device according to the moving speed and/or the moving path, automatically adjusts the output gear positions in accordance with the moving speed, so as to obtain same or similar body feeling when beauty care is performed at a single point, when the beauty care is performed during a rapid movement and when beauty care is performed in a section; furthermore, with respect to the beauty device having the multiple working modes, the working modes can be automatically adjusted according to the moving path, so as to satisfy diversified needs of the user.

### Embodiment 2

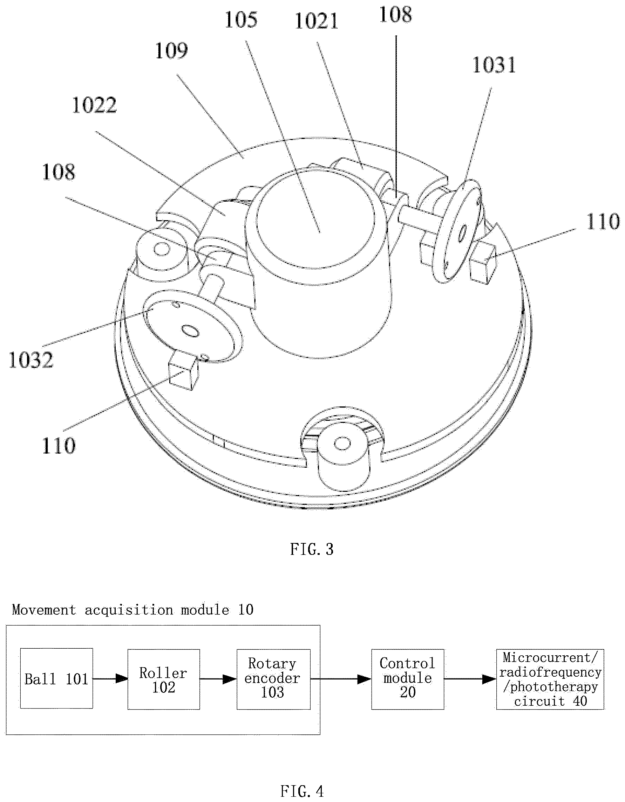

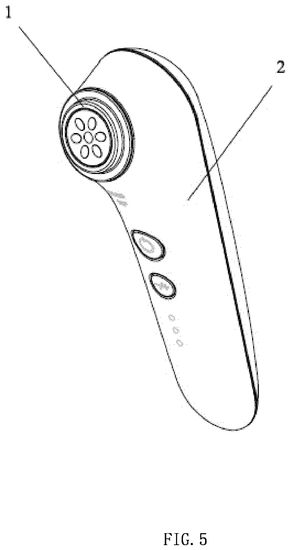

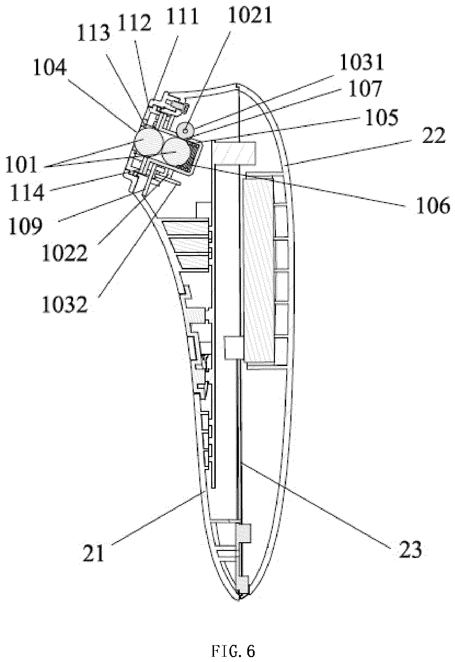

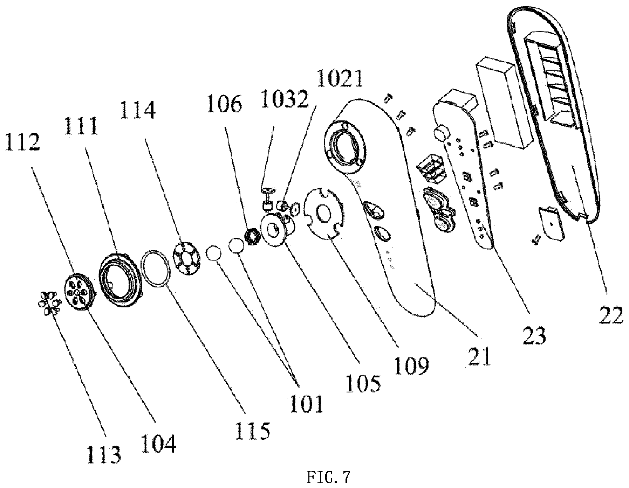

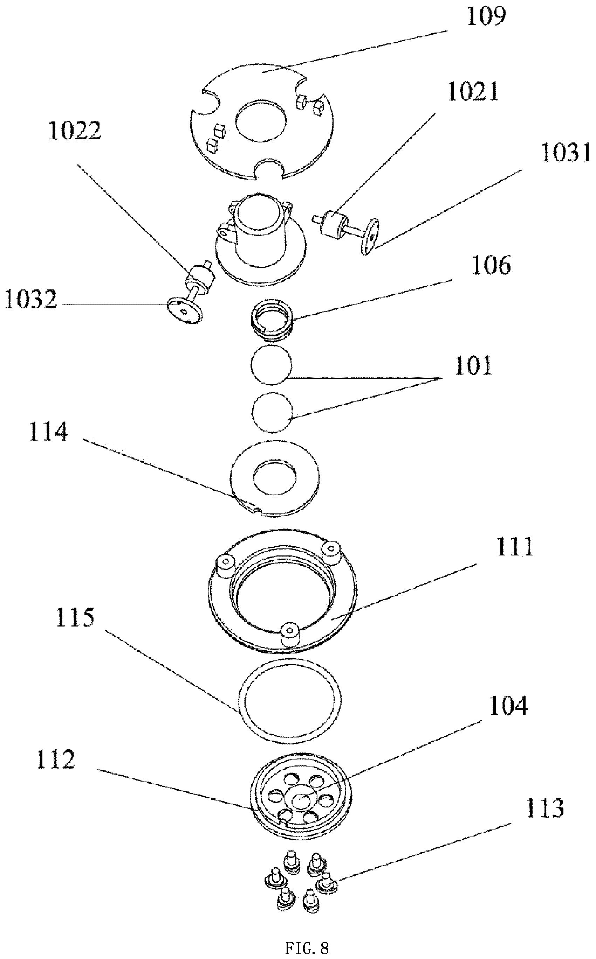

Referring to FIGS. 4 to 8, a facial beauty device of this embodiment comprises a handheld body 2 and a working head 1; it further comprises a control system of the facial beauty device based on one or more balls.

The control system of the facial beauty device based on the one or more balls comprises a movement acquisition module 10 and a control module 20; the movement acquisition module 10 comprises the one or more balls 101, one or more rollers 102, and one or more rotary encoders 103; the one or more balls 101 are disposed in the working head 1 of the facial beauty device and is partially exposed on a contact surface with skin; the one or more rollers 102 are connected to the one or more balls 101 by contacting so as to rotate when the one or more balls 101 roll; the one or more rollers 102 are coaxially connected to the one or more rotary encoders 103; the one or more rotary encoders 103 are electrically connected to the control module 20 to transmit pulse signals; the control module 20 receives the pulse signals to control a microcurrent/radiofrequency/phototherapy circuit 40 of the facial beauty device to regulate output gear positions and/or working modes.

In this embodiment the movement acquisition module 10 is disposed in the working head 1, and the control module 20 is disposed in the handheld body 2.

The facial beauty device of this embodiment integrates beauty care functions of microcurrent, phototherapy and RF radiofrequency. It should be noted that in other embodiments, the facial beauty device can merely comprise one of the beauty care functions of the microcurrent, the phototherapy and the RF radiofrequency, or comprise other beauty care functions and the present disclosure is specifically limited.

Specifically, the handheld body of this embodiment comprises an upper housing 21 and a lower housing 22, and the control module 20 is disposed in a cavity consisting of the upper housing 21 and the lower housing 22. The working head 1 of this embodiment comprises a fixed seat 111, a light-transmitting panel 112, a microcurrent/radiofrequency contact point 113, a microcurrent/radiofrequency/phototherapy PCB board 114 (a board on which the microcurrent/radiofrequency/phototherapy circuit 40 is located) and the movement acquisition module 10.

The light-transmitting panel 112 is connected to the microcurrent/radiofrequency/phototherapy PCB board 114 by passing through the fixed seat 111 and, and the light-transmitting panel 112 is fixedly connected to the fixed seat 111 by screws and the like. The microcurrent/radiofrequency/phototherapy PCB board 114 is connected to a main circuit board 23 (the main circuit board 23 comprises the control module 20, and the control module 20 comprises an MCU controller). The light-transmitting panel 112 comprises a first opening 104 for exposing the microcurrent/radiofrequency contact point 113. Radiofrequency, microcurrent and phototherapy massages of the facial beauty device is achieved through a combination of the light-transmitting panel 112, the microcurrent/radiofrequency contact point 113, the microcurrent/radiofrequency/phototherapy PCB board 114 and so on are existing techniques, and a realization principle and an operation principle thereof are not specifically described in this embodiment.

In this embodiment, the first opening 104 for exposing the one or more balls 101 is disposed on the light-transmitting panel 112. Specifically, the first opening 104 is disposed on a center of the light-transmitting panel 112.

In actual installation, the light-transmitting panel 112 is fixedly connected to the fixing seat 111, an accommodating cavity 105 of the movement acquisition module 10 is fixedly connected to the fixing seat 111, one end of the one or more balls 101 disposed in the accommodating cavity 105 passes through the fixing seat 111 to be exposed on the first opening 104 of the light-transmitting panel 112, and the other end abuts a bottom of the accommodating cavity 105 or be tightly pressed on a spring 106.

In this embodiment, a waterproof gasket 115 for waterproof is further disposed in the working head 1 to protect internal parts of the working head 1. Specifically, the waterproof washer 115 can be tightly pressed on the fixing seat 111.

The specific implementation of the movement acquisition module 10 and the control module 20 of this embodiment is the same as Embodiment 1 and will not be repeated herein.

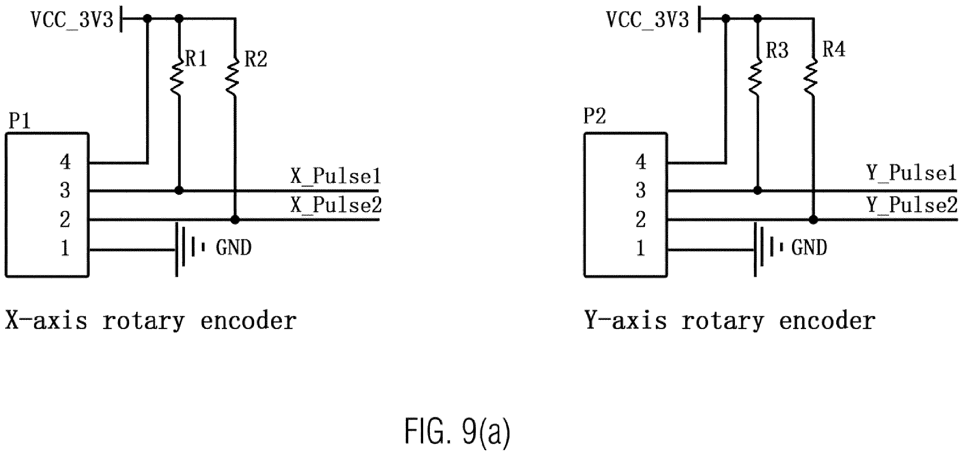

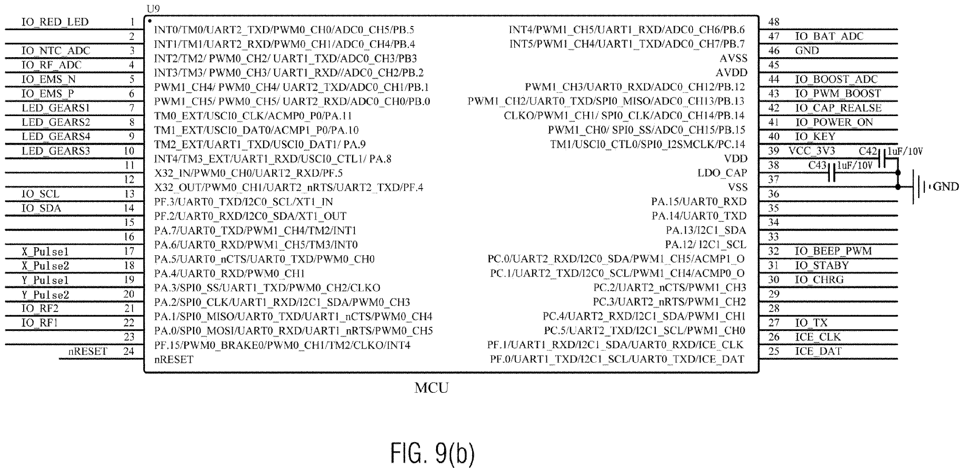

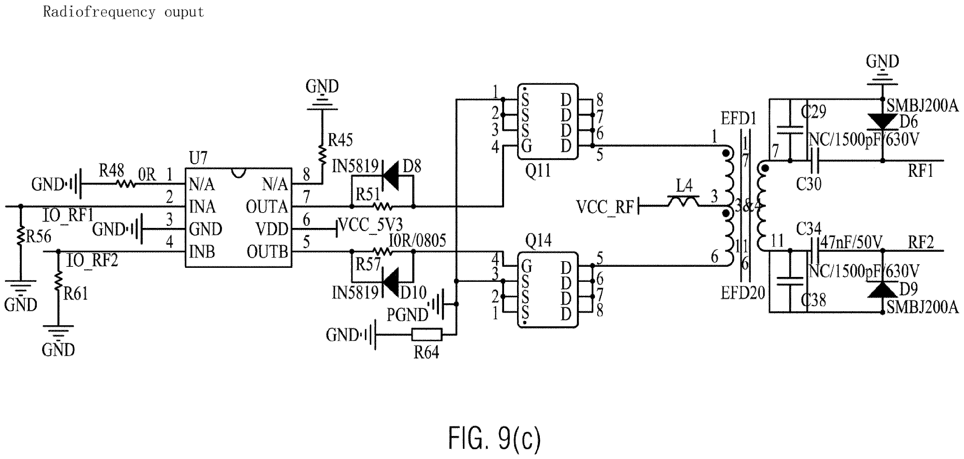

FIG. 9 shows a circuit diagram of the control system of the facial beauty device of this embodiment; wherein (a) shows a circuit diagram of an input of the one or more rotary encoders 103; (b) shows a circuit diagram of the MCU controller; and (c) shows a circuit diagram of an output of the radiofrequency. Specifically, the one or more rotary encoders 103 outputs the pulse signals (X-Pulse1, X-Pulse2/Y-Pulse1, Y-Pulse2) to the MCU controller, and the MCU controller outputs radiofrequency signals IO_RF1/ IO_RF1 to a radiofrequency module.

In this embodiment, the control module 20 recognizes that the moving speed of the one or more balls 101 increases and is greater than a preset value or lasts a preset time, which indicates that the facial beauty device is moving rapidly, the output gear positions of the facial beauty device can be automatically adjusted to a higher gear position (specifically, a temperature of the radiofrequency increases, a magnitude of the microcurrent increases, an energy level of the phototherapy increases, etc.), and no further adjustment is performed when the highest gear position is reached; when the control module 20 recognizes that the moving speed of the one or more balls 101 slows down and is less than the preset value or lasts the preset time, which indicates that the facial beauty device is decelerated, the output gear positions of the facial beauty device can be automatically adjusted to a lower gear position (specifically, the temperature of the radiofrequency decreases, the magnitude of the microcurrent decreases, the energy level of the phototherapy decreases, etc.); when the control module 20 recognizes that the moving speed of the one or more balls 101 is 0 and lasts the preset time, the gear positions can be automatically adjusted to the lowest gear position, further, when it recognizes that the moving speed of the one or more balls 101 is 0 and lasts another preset time more than the preset time, in order to prevent a motor from burning out due to blocking of the working head 1, a power supply can be automatically shut down and prompted.

Specifically, a principle for adjusting a strength of the gear positions of the facial beauty device in a moving process is that different speed intervals correspond to strength values of different gear positions, specifically:
A lowest value of power output is set as Pmin, Pmin corresponds to a speed of Vmin; a highest value of the power output is set as Pmax, Pmax corresponds to a speed of Vmax; when a current detected moving speed of the facial beauty device is Vnow, a power corresponding to the current speed is Pv = Pmin + (Vnow * (Pmax-Pmin)) / (Vmax-Vmin). Variations in a power level correspond to different radiofrequency temperatures, microcurrent magnitudes, phototherapy energy levels, etc.

Further, in the facial beauty device of this embodiment, if a ratio of the moving distance in an X-axis and the moving distance in a Y-axis shows a linear variation with a relatively fixed slope, the moving path is judged to be a straight movement; if the moving distance in the X-axis and the moving distance in the Y-axis define alternate increasing and decreasing variations, the moving path is judged to be a circular movement. In a straight reciprocating movement, simultaneous output are achieved by switching to the radiofrequency and red light to perform heating and massage in a wide range to accelerate blood circulation; in the circular movement in a small range, considering a range of use is mainly an eye corner, a mouth corner and other areas, a mode in which the radiofrequency and the microcurrent are alternately output is used to stimulate cellular activity and promote collagen fibers to contract.

It should be noted that if a variation of the moving path is detected in a fixed gear position, the working modes are adjusted, similarly, if a variation of the moving speed is detected in a certain working mode, the output gear positions are adjusted, i.e., the working modes and the output gear positions can be alternately adjusted.

### Embodiment 3

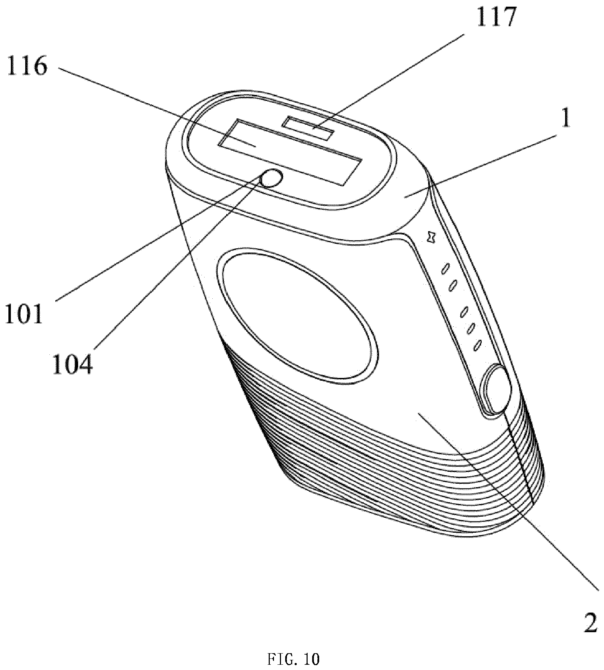

Referring to FIGS. 1, 2, 3 and 10, a hair removal device of this embodiment comprises a handheld body 2 and a working head 1; and it further comprises a control system of the hair removal device based on one or more balls.

The control system of the hair removal device based on the one or more balls comprises: a movement acquisition module 10 and a control module 20; the movement acquisition module 10 comprises one or more balls 101, one or more rollers 102 and one or more rotary encoders 103; the one or more balls 101 are disposed in the working head 1 of the hair removal device and is partially exposed on a contact surface with a skin; the one or more rollers 102 are connected to the one or more balls 101 by contacting so as to rotate when the one or more balls 101 rolls; the one or more rollers 102 are coaxially connected to the one or more rotary encoders 103; the one or more rotary encoders 103 are electrically connected to the control module 20 to transmit pulse signals; the control module 20 receives the pulse signals to control an output circuit 30 of the hair removal device of a beauty device to regulate output gear positions and/or working modes, specifically, optical densities/energy levels, etc., of the hair removal device is regulated.

Components, positions thereof and connection relationships thereof of the control system of the hair removal device based on the one or more balls are the same as the control system of the beauty device and are not described in this embodiment.

The movement acquisition module 10 in this embodiment is disposed in the working head 1, and the control module 20 is disposed in the handheld body 2.

The hair removal device of this embodiment comprises functions of hair removal and skin detection, etc. It should be noted that in other embodiments, the hair removal device can merely have a hair removal function, and the present disclosure does not specifically limited.

Specifically, the working head 1 of this embodiment comprises a light outlet 116, a skin detection point 117 and a first opening 104 for exposing the one or more balls 101.

The specific implementation of the movement acquisition module 10 and the control module 20 of this embodiment is the same as that of Embodiment 1 and will not be repeated herein.

In this embodiment, when the control module 20 recognizes that the moving speed of the one or more balls 101 increases and is greater than a preset value or lasts a preset time, which indicates that the hair removal device is moving rapidly, the output gear positions of the hair removal device can be automatically adjusted to a higher gear position (specifically, the optical densities/the energy levers, etc., of the hair removal device increase), and no further adjustment is performed when the highest gear position is reached; when the control module 20 recognizes that the moving speed of the one or more balls 101 slows down and is less than the preset value or lasts the preset time, which indicates that the hair removal device is decelerated, the output gear positions of the hair removal device can be automatically adjusted to a lower gear position (specifically, the optical densities/the energy levers, etc., of the hair removal device decrease); when the control module 20 recognizes that the moving speed of the one or more balls 101 is 0 and lasts the preset time, the gear positions can be automatically adjusted to the lowest gear position, further, when it recognizes that the moving speed of the one or more balls 101 is 0 and lasts another preset time more than the preset time, in order to prevent a motor from burning out due to blocking of the working head 1, a power supply can be automatically shut down and prompted.

Specifically, a principle for adjusting a strength of the gear positions of the hair removal device in a moving process is that different speed intervals correspond to strength values of different gear positions, specifically:
A lowest value of power output is set as Pmin, Pmin corresponds to a speed of Vmin; a highest value of the power output is set as Pmax, Pmax corresponds to a speed of Vmax; when a current detected moving speed of the hair removal device is Vnow, a power corresponding to the current speed is Pv = Pmin + (Vnow* (Pmax-Pmin)) / (Vmax-Vmin). Variations in a power level correspond to different optical densities/energy levels, etc.

The aforementioned embodiments are merely preferred embodiments of the present disclosure, and the scope of the disclosure is not limited thereto. Thus, it is intended that the present disclosure cover modifications and variations provided they are made within the technical scope disclosed by the present disclosure based on the technical solutions and improved concepts of the present disclosure by any technical person skilled in the art.

### INDUSTRIAL APPLICABILITY

The present disclosure discloses a control system of a beauty device and the beauty device, a movement acquisition module thereof is used to convert a movement signal of the beauty device during work into an electrical signal, the control module is connected to the movement acquisition module, receives the electrical signal, obtains movement data of the beauty device by analyzing, and controls an output gear position and/or a working mode of the beauty device based on the movement data. The present disclosure can be used to various beauty devices, detect variations of a movement state of the beauty device, adjust the output gear position and/or the working mode to improve user experience, and has good industrial applicability.

## Claims

1. A control system of a beauty device, **characterized in that**, comprising: a movement acquisition module and a control module; the movement acquisition module is used to convert a movement signal of the beauty device during work into an electrical signal; the control module is connected to the movement acquisition module, receives the electrical signal, obtains movement data of the beauty device by analyzing, and controls an output gear position and/or a working mode of the beauty device based on the movement data.

2. The control system of the beauty device according to claim 1, **characterized in that**, the electrical signal comprises a pulse signal; the movement acquisition module is used to convert the movement signal of the beauty device during work into the electrical signal, specifically comprising:
the movement acquisition module is used to convert the movement signal of the beauty device during work into a rolling signal and output the pulse signal based on the rolling signal.

3. The control system of the beauty device according to claim 2, **characterized in that**, the movement acquisition module comprises one or more balls, one or more rollers and one or more rotary encoders; the one or more balls are disposed in a working head of the beauty device and are partially exposed on a contact surface with a skin; the one or more rollers are connected to the one or more balls by contacting so as to rotate when the one or more balls roll; the one or more rollers are coaxially connected to the one or more rotary encoders; the one or more rotary encoders are electrically connected to the control module to transmit the pulse signal; the control module receives the pulse signal to control the output gear position and/or the working mode of the beauty device.

4. The control system for the beauty device according to claim 3, **characterized in that**, the working head comprises a first opening for exposing the one or more balls.

5. The control system of the beauty device according to claim 3, **characterized in that**, the one or more rollers comprise an X-axis roller for detecting a movement of the one or more balls in an X-axis and a Y-axis roller for detecting a movement of the one or more balls in a Y-axis; the one or more rotary encoders comprise an X-axis rotary encoder and a Y-axis rotary encoder; the X-axis roller is connected to the X-axis rotary encoder; the Y-axis roller is connected to the Y-axis rotary encoder; the X-axis rotary encoder and Y-axis rotary encoder are respectively electrically connected to the control module.

6. The control system of the beauty device according to claim 3, **characterized in that**, the movement acquisition module further comprises an accommodating cavity; the one or more balls are disposed in the accommodating cavity.

7. The control system of the beauty device according to claim 6, **characterized in that**, the one or more balls disposed in the accommodating cavity comprise one or more; when two or more are included, all of the one or more balls are disposed by following a direction of a bottom of the accommodating cavity in sequence.

8. The control system of the beauty device according to claim 6, **characterized in that**, a spring is disposed on the bottom of the accommodating cavity; the spring is compressed by the one or more balls in the accommodating cavity.

9. The control system of the beauty device according to claim 6, **characterized in that**, a side of the accommodating cavity comprises one or more second openings; the one or more balls are in close contact with the one or more rollers through the one or more second openings.

10. The control system of the beauty device according to claim 6, **characterized in that**, an outer side of the accommodation cavity is disposed with lugs for securing the one or more rollers.

11. The control system of the beauty device according to claim 2, **characterized in that**, the control module receives the pulse signal, obtains the movement data of the beauty device by analyzing, and controls the output gear position of the beauty device based on the movement data, specifically comprising:
the control module receives the pulse signal and detects a number of received pulses within a preset time;
the number of the received pulses is converted into a moving distance, the moving distance is divided by the preset time to obtain a moving speed of the one or more balls;
the output gear position of the beauty device is adjusted based on the moving speed of the one or more balls.

12. The control system of the beauty device according to claim 5, **characterized in that**, the control module receives the pulse signal, obtains the movement data of the beauty device by analyzing, and controls the working mode of the beauty device based on the movement data, specifically comprising:
the control module receives the pulse signal and detects a number of pulses in the X-axis and a number of pulses in the Y-axis received within a preset time;
the number of the pulses in the X-axis is converted into a moving distance in the X-axis; the pulse number in the Y-axis is converted into a moving distance in the Y-axis;
a moving path of the one or more balls is detected based on the moving distance in the X-axis and the moving distance in the Y-axis;
the working mode of the beauty device is adjusted based on the moving path of the one or more balls.

13. A beauty device comprising a handheld body and a working head; **characterized in that**: it further comprises the control system of the beauty device according to any one of claims 1 to 12; the movement acquisition module is disposed in the working head;
the movement acquisition module comprises one or more balls, one or more rollers and one or more rotary encoders; the one or more balls are disposed in the working head of the beauty device and are partially exposed on a contact surface with a skin; the one or more rollers are connected to the one or more balls by contacting so as to rotate when the one or more balls roll; the one or more rollers are coaxially connected to the one or more rotary encoders; the one or more rotary encoders are electrically connected to the control module to transmit a pulse signal;
the one or more rollers comprise an X-axis roller for detecting a movement of the one or more balls in an X-axis and a Y-axis roller for detecting a movement of the one or more balls in a Y-axis; the one or more rotary encoders comprise an X-axis rotary encoder and a Y-axis rotary encoder;
the control module receives the pulse signal, obtains the movement data of the beauty device by analyzing: the output gear position of the beauty device is adjusted based on a moving speed of the one or more balls; the working mode of the beauty device is adjusted based on a moving path of the one or more balls.

14. The beauty device according to claim 13, **characterized in that**, the beauty device comprises a facial beauty device.

15. The beauty device according to claim 13, **characterized in that**, the beauty device comprises a hair removal device.
